# EUROPEAN PATENT APPLICATION

(11) **EP 4 206 323 A1**
(43) Date of publication of application: **05.07.2023**
(21) Application number: 21861724.9
(22) Date of filing: 27.08.2021
(51) Int. Cl.: C12N 15/10, C12Q 1/6806

(54) **RNA STORAGE METHOD**

(30) Priority: 28.08.2020 JP 2020144317
(71) Applicant: Kao Corporation, Chuo-ku Tokyo 103-8210 (JP)
(72) Inventor: NAGAMORI, Natsumi, Haga-gun, Tochigi 321-3497 (JP); INOUE, Takayoshi, Haga-gun, Tochigi 321-3497 (JP); UEHARA, Yuya, Haga-gun, Tochigi 321-3497 (JP); OYA, Naoki, Odawara-shi, Kanagawa 250-0002 (JP)
(74) Representative: Vossius & Partner Patentanwälte Rechtsanwälte mbB
(86) International application number: PCT/JP2021/031568
(87) International publication number: WO 2022/045303

(57) **Abstract**

The present invention provides a method for preventing degradation of skin surface lipid-derived RNA, comprising treating a collection medium containing skin surface lipids with an aqueous solution which comprises at least one selected from the group consisting of guanidine hydrochloride and guanidine thiocyanate and in which water or an alcohol aqueous solution is used as a solvent.

## Description

### Field of the Invention

The present invention relates to an RNA storage method.

### Background of the Invention

Through analysis of nucleic acids derived from an organism such as DNA and RNA, various information including the physiological state of the organism can be acquired. A nucleic acid derived from an organism can be extracted from a sample derived from an organism such as a cell, a tissue, a body fluid, and a secretion. Patent Literature 1 describes that skin surface lipids (SSL) contain RNA, and the SSL-derived RNA can be used for analysis of an organism. The amount of RNA contained in SSL is, however, very small, and hence, it is desired to extract RNA in a yield as high as possible from SSLs collected from an organism.

Among nucleic acids derived from an organism, RNA is unstable and easily degraded as compared with DNA. Ribonuclease (RNase), that is, an RNA-degrading enzyme, is widely present not only in a cell and a tissue but also in a body fluid such as sweat or saliva, and under the environment. For preventing degradation of RNA in a biological sample, a method in which RNA is immediately extracted from a collected sample, a method in which RNA present in or extracted from a sample is stored at -80°C, a method in which an RNase inhibitor is used, and the like have been conventionally employed. For example, when a chaotropic salt such as a guanidine salt is added to a biological sample, a protein containing RNase included in the sample can be denatured to inactivate the RNase. Even when a chaotropic salt is added, however, RNA degradation may not be completely prevented at ambient temperature, and therefore, RNA present in or extracted from a sample is usually stored at -80°C.

Patent Literature 2 describes a simple extraction method for nucleic acid including a step of adding, to a biological sample, a protein denaturant such as guanidine thiocyanate, guanidine hydrochloride, potassium thiocyanate, sodium thiocyanate, or urea, and disrupting a cell membrane, a cell wall or the like, followed by protein denaturation to thereby releasing a nucleic acid alone; a step of adding an alcohol to thereby aggregate the released nucleic acid, a step of allowing the resultant nucleic acid-containing extract to pass through a filter to thereby capture the nucleic acid with the filter, and a step of solubilizing the nucleic acid with purified water or a low concentration buffer to thereby collect the nucleic acid from the filter.

Patent Literature 3 describes that an RNA sample obtained from milk can be stored for a long period of time of 3 days or more by extracting RNA from milk with an RNA extraction agent containing phenol and guanidine thiocyanate, and then rapidly freezing the RNA at -80°C.

Patent Literature 4 describes a method for extracting nucleic acid from a sample and storing it, including collecting nucleic acid from the sample by applying a solid matrix in which a composition containing a protein denaturant such as guanidine hydrochloride, guanidium thiocyanate, arginine, SDS, or urea, a reducing agent, and a buffer is present in a dry state, drying the solid matrix, and storing the nucleic acid on the solid matrix in a dry state under ambient conditions.
(Patent Literature 1) WO 2018/008319
(Patent Literature 2) JP-A-2009-153467
(Patent Literature 3) JP-A-2000-264898
(Patent Literature 4) JP-A-2018-68303

### Summary of the Invention

The present invention provides a method for preventing degradation of skin surface lipid-derived RNA, comprising:
treating a collection medium containing skin surface lipids with an aqueous solution which comprise at least one selected from the group consisting of guanidine hydrochloride and guanidine thiocyanate and in which water or an alcohol aqueous solution is used as a solvent.

The present invention also provides a method for storing skin surface lipid-derived RNA, comprising:
treating a collection medium containing skin surface lipids with an aqueous solution which comprises at least one selected from the group consisting of guanidine hydrochloride and guanidine thiocyanate and in which water or an alcohol aqueous solution is used as a solvent; and
storing the treated collected medium.

The present invention also provides a kit for collecting skin surface lipid-derived RNA, comprising a collection medium for collecting skin surface lipids, and an aqueous solution for treating the collection medium which comprises at least one selected from the group consisting of guanidine hydrochloride and guanidine thiocyanate and in which water or an alcohol aqueous solution is used as a solvent.

The present invention also provides a method for recovering skin surface lipid-derived RNA of a subject, comprising:
providing, to a subject needing or desiring collection of skin surface lipid-derived RNA, the above-described kit for collecting skin surface lipid-derived RNA the kit comprising a collection medium for collecting skin surface lipids, and an aqueous solution for treating the collection medium,
wherein the aqueous solution comprises at least one selected from the group consisting of guanidine hydrochloride and guanidine thiocyanate in which water or an alcohol aqueous solution is used as a solvent; and
recovering, from the subject, the collection medium containing skin surface lipids collected from the subject,
wherein the recovered collection medium containing the skin surface lipids has been treated with the aqueous solution in accordance with the above-described method for preventing degradation of or for storing skin surface lipid-derived RNA, and has been stored at ambient temperature after collecting the skin surface lipids of the subject.

### Brief Description of Drawings

[Figure 1] Figure 1 is a diagram illustrating an example of a container used for storing a collection medium in a kit for collecting SSL-derived RNA of the present invention, A: a side view (on a left side) and a cross-sectional view thereof (on a right side), and B: a perspective view from above (on a left side) and a cross-sectional view thereof (on a right side).
[Figure 2] Figure 2 is a diagram illustrating an example of the container of Figure 1 in a state where a collection medium containing SSL-derived RNA and a desiccant are held therein.

### Detailed Description of the Invention

Patent literatures, non-patent literatures, and other publications cited herein are all incorporated herein by reference.

As used herein, the term "RNA" encompasses single-stranded RNA and double-stranded RNA, such as mRNA, tRNA, rRNA, small RNA (such as microRNA (miRNA), small interfering RNA (siRNA), or PIWI-interacting RNA (piRNA)), long intergenic non-coding (line) RNA, and the like.

The term "skin surface lipids (SSL)" used herein refers to a lipid-soluble fraction present on the surface of the skin, and is also designated as sebum. In general, SSL mainly contains a secretion secreted from an exocrine gland such as a sebaceous gland in the skin, and is present on the skin surface in the form of a thin layer covering the skin surface.

The term "skin" used herein is a generic term for a region on the body surface including tissues such as an epidermis, a dermis, a hair follicle, and a sweat gland, a sebaceous gland, and other glands unless especially specified.

The term "ambient temperature" used herein refers to a temperature under an environment where SSL can be collected, and preferably refers to room temperature or a temperature lower than it. Here, room temperature refers to a temperature of from 1 to 30°C defined in the Japanese Pharmacopoeia.

The present invention relates to a method for preventing degradation of RNA contained in skin surface lipids (SSL) collected from a subject, and stably storing the RNA.

The present inventors found that after collecting SSL from a subject with a collection medium, when the collection medium containing the SSL is treated with an aqueous solution which contains at least one selected from the group consisting of guanidine hydrochloride and guanidine thiocyanate and in which water or an alcohol aqueous solution is as a solvent, degradation of RNA contained in the SSL can be prevented, and the RNA can be stably stored.

In one aspect, the present invention provides a method for preventing degradation of SSL-derived RNA, and a method for storing SSL-derived RNA. Each of these methods of the present invention includes treating a collection medium containing SSL collected from a subject with an aqueous solution which contains at least one selected from the group consisting of guanidine hydrochloride and guanidine thiocyanate and in which water or an alcohol aqueous solution is used as a solvent.

According to the method of the present invention, degradation of RNA contained in SSL collected from a subject can be prevented, and the RNA can be stably stored. According to the present invention, RNA, which is easily degraded and is difficult to handle, can be stored at ambient temperature. According to the present invention, an RNA yield from SSL, and accuracy of various analyses using the RNA can be improved.

In the method of the present invention, a subject from which SSL is collected may be any living thing having SSL on the skin. Examples of the subject include mammals including a human and a non-human mammal, and the subject is preferably a human. For example, the subject can be a human or a non-human mammal which needs or desires analysis of its own nucleic acid. Alternatively, the subject can be a human or a non-human mammal which needs or desires gene expression analysis on the skin, or analysis, using nucleic acid, of a state of the skin or a part excluding the skin.

Examples of the part of the skin from which SSL of the subject is collected include, but are not especially limited to, skins in arbitrary body parts including the head, the face, the neck, the trunk, the limb and the like, such as a healthy skin, a skin having a disease such as atopy, acne, dryness, inflammation (redness), and a tumor, and a wounded skin.

Examples of the collection medium to be used for collecting SSL from the skin of a subject include an SSL-absorbent material. Examples of the SSL-absorbent material include a material which has a fibrous structure or a porous structure, and can collect and support SSL in a gap of the structure by capillary action, a material capable of adsorbing SSL with a lipophilic material, and a material having both of these properties. Examples of a raw material of the SSL-absorbent material include plastics such as polypropylene, pulp, cellulose, cotton, synthetic fiber such as rayon, and the like, and the material is preferably polypropylene having a porous structure.

From the viewpoints of efficiency of collecting SSL, and efficiency of drying of the collection medium described below, the collection medium is more preferably in a shape having a larger ratio of surface area to volume. The collection medium is preferably in a sheet shape.

Specific examples of the collection medium include a porous plastic film, paper, gauze, and a cotton ball, and commercially available oil absorbing paper or oil absorbing film can be used. Preferable examples of the collection medium include a porous plastic film having a film thickness of from 5 to 200 µm, a porosity of from 5 to 50%, and a pore diameter of from 0.2 to 5 µm, and an oil absorbing cosmetic sheet made of a plastic porous stretched film described in JP-B-3055778 (such as Oil Clear Film (Hakugen Earth Company, Limited.) using a porous polypropylene film available from 3M). It is noted that the porous plastic film may be subjected to a hydrophilic treatment as long as SSL absorption is not inhibited.

In order to improve the efficiency of collecting SSL, a collection medium precedently impregnated with a solvent having a high lipid solubility may be used. In contrast, a collection medium containing a solvent having a high water solubility or a moisture is not preferable because SSL adsorption is inhibited. The collection medium is used preferably in a dry state.

In the method of the present invention, the collection medium containing SSL collected from a subject is treated with an aqueous solution containing at least one selected from the group consisting of guanidine hydrochloride and guanidine thiocyanate (hereinafter referred to as an aqueous treatment solution). The aqueous treatment solution preferably contains an alcohol. The aqueous treatment solution preferably contains guanidine hydrochloride. The aqueous treatment solution can be prepared by dissolving at least one selected from the group consisting of guanidine hydrochloride and guanidine thiocyanate in water or an aqueous solution containing an alcohol used as a solvent. Examples of alcohol which can be contained in the alcohol aqueous solution used as the solvent include one or more selected from the group consisting of ethanol, isopropanol, and butanol, and from the viewpoint of efficiency of drying of the collection medium described below, ethanol is preferably used.

An alcohol concentration in the alcohol aqueous solution used as the solvent is not especially limited, and from the viewpoints of permeability into the SSL-absorbent material, and efficiency of drying of the collection medium described below, the alcohol concentration is preferably 20% by volume or more, more preferably 30% by volume or more, and further more preferably 50% by volume or more, and on the other hand, from the viewpoint of safety in handling the aqueous solution, is preferably 70% by volume or less. The alcohol concentration in the alcohol aqueous solution used as the solvent is preferably 20% by volume or more and 70% by volume or less, more preferably 30% by volume or more and 70% by volume or less, further more preferably 50% by volume or more and 70% by volume or less, and further more preferably 50% by volume or more and less than 60% by volume. It is noted that the term "volume" used here means the volume at 25°C.

A content, in the aqueous treatment solution, of the at least one selected from the group consisting of guanidine hydrochloride and guanidine thiocyanate (a total amount of guanidine hydrochloride and guanidine thiocyanate) is preferably 0.50 g/mL or more, and more preferably 0.55 g/mL or more from the viewpoint of prevention of RNA degradation, and on the other hand, may be equal to or lower than a saturation concentration in the solvent of the aqueous treatment solution.

In the treatment of the collection medium with the aqueous treatment solution, SSL contained in the collection medium is contacted with the aqueous treatment solution. For example, the treatment includes dropping the aqueous treatment solution onto the collection medium, applying the aqueous treatment solution to the collection medium, spraying the aqueous treatment solution onto the collection medium, contacting the collection medium with a support that supports the aqueous treatment solution to allow the aqueous treatment solution to permeate the collection medium via the support, or dipping a part of the collection medium in the aqueous treatment solution. The aqueous treatment solution permeates the collection medium by any of these methods. The aqueous treatment solution may permeate the whole collection medium, but may permeate at least a portion containing SSL of the collection medium.

An amount of the aqueous treatment solution used in the treatment of the collection medium is preferably such an amount that it permeates at least the portion containing SSL of the collection medium. For example, when the collection medium is a sheet-shaped collection medium, the amount of the aqueous treatment solution to be used is preferably such an amount that the aqueous treatment solution permeates 80% or more of the sheet area. Alternatively, the amount of the aqueous treatment solution is preferably such an amount that it can be supported in or on the collection medium. When the amount of the aqueous treatment solution to be applied is too small, RNA unavoidably degrades in a portion where the solution does not permeate. In contrast, when the amount of the aqueous treatment solution to be applied is too large, it is apprehended that SSL contained in the collection medium may flow out of the collection medium together with the aqueous treatment solution. When the amount of the aqueous treatment solution to be applied is too large, it is apprehended that it may take a long time to dry the collection medium treated with the aqueous treatment solution or a sufficient dry state is obtained in the drying described below. In consideration of these factors, the amount of the aqueous treatment solution to be applied to the collection medium may be appropriately adjusted depending on the type and size of the collection medium.

The method of the present invention preferably further includes drying the collection medium treated with the aqueous treatment solution. When the collection medium is dried, RNA degradation with RNase, that is, a hydrolase, is more effectively prevented. Methods for drying the collection medium is not especially limited as long as the collection medium can be placed under a dry environment with the methods, and from the viewpoint of preventing RNase contamination, it is preferable to place the collection medium under a dry environment in a closed space system such as in a container. Specifically, when the collection medium is caused to coexist with a desiccant in a closed space system such as in a container capable of holding the collection medium therein, the drying of the collection medium can be accelerated to more effectively prevent RNA degradation. The container is not especially limited in size and shape as long as it is made of a material which water does not permeate, can hold the collection medium and the desiccant therein, and can be sealed. In consideration that heat may be generated through contact between the aqueous treatment solution contained in the collection medium and the desiccant, it is preferable that the container has a structure or configuration in which the collection medium and the desiccant are not in direct contact with each other. Examples of the desiccant include crystalline zeolite (molecular sieves), activated carbon, and silica gel. An amount of the desiccant to be used is preferably adjusted such that the closed space holding the collection medium therein can be placed under a dry environment (preferably with a humidity reduced to 0%). For example, molecular sieves in an amount of 0.05 to 0.10 g per cm³ of the closed space are preferably used. Alternatively, when the collection medium is a sheet-shaped collection medium, molecular sieves in an amount of 0.05 to 0.20 g per cm² of the sheet area are preferably used. Alternatively, molecular sieves in an amount of 0.02 to 0.10 g per µL of the aqueous treatment solution permeating the collection medium are preferably used.

The collection medium obtained through the above-described procedures, by the treatment with the aqueous treatment solution, and preferably further the drying, can be stored as a sample containing SSL-derived RNA. Through the treatment with the aqueous treatment solution and the drying process, the degradation of the SSL-derived RNA contained in the collection medium is prevented, and hence the thus obtained collection medium can be stored at ambient temperature, or a temperature higher than it (of, for example, 37°C or less, 40°C or less, or 60°C or less).

In another aspect, the present invention provides a kit for collecting SSL-derived RNA. The kit of the present invention includes the above-described collection medium for collecting SSL, and as an aqueous treatment solution for treating the collection medium, the aqueous solution which contains at least one selected from the group consisting of guanidine hydrochloride and guanidine thiocyanate and in which water or an alcohol aqueous solution is as a solvent. In one embodiment, the kit further includes a container for storing the collection medium containing SSL treated with the aqueous treatment solution. The container is sealable, and can be used for drying or storing the collection medium treated with the aqueous treatment solution. The container of the kit is preferably used in combination with a desiccant. Accordingly, the kit preferably includes the container and a desiccant. For example, when the collection medium treated with the aqueous treatment solution is put in the container to be caused to coexist with the desiccant held in the container, and the container is sealed, the drying of the collection medium in the container is accelerated. The collection medium thus dried can be stored directly in the container. The container holding the collection medium therein can be stored at ambient temperature, or a temperature higher than it (of, for example, 37°C or less, 40°C or less, or 60°C or less).

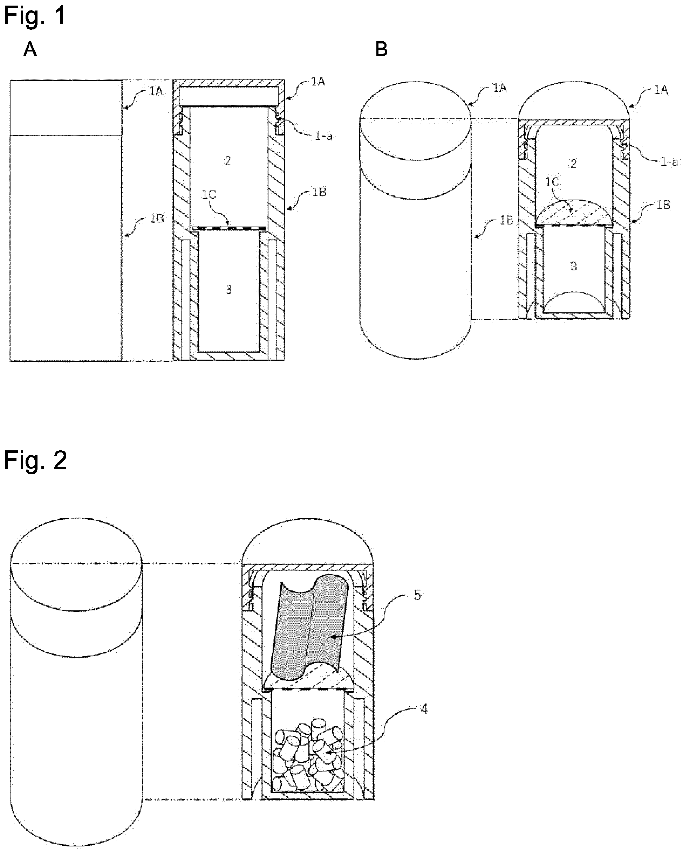

Figure 1 illustrates an example of the container for storing the collection medium used in the kit of the present invention. In Figure 1, A illustrates a side view (on a left side) and a cross-sectional view thereof (on a right side), and B illustrates a perspective view from above (on a left side) and a cross-sectional view thereof (on a right side). The container is composed of a cap 1A of the container and a container body 1B, and in a part 1-a where the cap 1A and the container body 1B are in contact with each other, spiral ribs are provided on the inner surface of the cap 1A and on the outer surface of the container body 1B, and a closed space can be formed inside the container when the cap 1A and the container body 1B are screwed together. The inside of the container is partitioned with a member 1C of a breathable material or structure into a storage room 2 and a storage room 3. A collection medium after SSL collection treated with the aqueous treatment solution and a desiccant are respectively held in the respective storage rooms, and thus, these can be held in the same closed space within the container without coming into contact with each other. In the container, the desiccant is preferably precedently held in the container. Figure 2 illustrates an example of the container of Figure 1 in a state where the collection medium containing SSL-derived RNA and a desiccant are held therein. The desiccant 4 is held in the storage room 3 of the container, and the collection medium 5 after the SSL collection treated with the aqueous treatment solution is held in the storage room 2 partitioned by the breathable member 1C. The closed space in the whole inside of the container is placed under a dry environment through the breathable member 1C, hence the drying of the collection medium is accelerated, and thus, RNA can be stored at ambient temperature, or a temperature higher than it (of, for example, 37°C or less, 40°C or less, or 60°C or less) .

The collection medium treated by the method of the present invention can be used as a sample containing SSL-derived RNA. Extraction of RNA from the collection medium can be performed in accordance with usual procedures for RNA extraction from a sample. For example, RNA can be extracted from the collection medium in accordance with such as a phenol/chloroform method or a modified method thereof, such as PCI (phenol/chloroform/isoamyl alcohol) method, AGPC (acid guanidine thiocyanate-phenol-chloroform extraction) method, or AGPC modified method in which guanidine thiocyanate and phenol are precedently mixed. Alternatively, RNA can be extracted from the collection medium with a commercially available RNA extraction reagent (such as TRIzol(R) Reagent, QIAzol Lysis Reagent, ISOGEN, or RNeasy Mini Kit).

The RNA thus extracted is SSL-derived RNA of the subject, and can be used in various analyses. For example, mRNA contained in the SSL-derived RNA is converted to cDNA with an Oligo(dT) primer, and then the resultant can be used for gene expression analysis, transcriptome analysis or the like. Alternatively, functional analysis of the subject, diagnosis of a disease, evaluation of efficacy of a drug administered to the subject, and the like can be performed by checking whether or not a target RNA is present in the SSL-derived RNA of the subject.

In another aspect, the present invention provides a method for recovering skin surface lipid-derived RNA of a subject. The method of the present invention includes providing, to a subject needing or desiring collection of skin surface lipid-derived RNA, the above-described kit for collecting skin surface lipid-derived RNA of the present invention, and recovering, from the subject, the collection medium containing skin surface lipids collected from the subject by using the collection medium included in the kit. Here, the collection medium containing the skin surface lipids recovered from the subject has been treated with the aqueous solution in accordance with the above-described method for preventing degradation of or for storing skin surface lipid-derived RNA of the present invention. The collection medium containing the skin surface lipids recovered from the subject is stored at ambient temperature, or a temperature higher than it (of, for example, 37°C or less, 40°C or less, or 60°C or less) after collecting the skin surface lipids of the subject. Here, a method for providing the kit for collecting skin surface lipid-derived RNA, and a method for recovering the collection medium from the subject after collecting skin surface lipids are not especially limited, and known transportation means can be employed, and examples thereof include such as postal service, courier service by a freight forwarder, receipt by the subject or a representative at a distribution base of the collection kit, and bringing to a recovery base of the collection medium by the subject or a representative.

As exemplified embodiments of the present invention, the following substances, production methods, uses, methods and the like are further disclosed herein. It is noted that the present invention is not limited by these embodiments.
[1] A method for preventing degradation of skin surface lipid-derived RNA, comprising:
   treating a collection medium containing skin surface lipids with an aqueous solution which comprises at least one selected from the group consisting of guanidine hydrochloride and guanidine thiocyanate and in which water or an alcohol aqueous solution is used as a solvent.
[2] A method for storing skin surface lipid-derived RNA, comprising:
   treating a collection medium containing skin surface lipids with an aqueous solution which comprises at least one selected from the group consisting of guanidine hydrochloride and guanidine thiocyanate and in which water or an alcohol aqueous solution is used as a solvent; and
   storing the treated collection medium.
[3] The method according to [1] or [2], preferably further comprising drying the collection medium treated with the aqueous solution.
[4] The method according to any one of [1] to [3], in which the solvent is preferably an alcohol aqueous solution, and an alcohol concentration in the alcohol aqueous solution is
   preferably 20% by volume or more,
   more preferably 20% by volume or more and 70% by volume or less,
   further more preferably 30% by volume or more,
   further more preferably 30% by volume or more and 70% by volume or less,
   further more preferably 50% by volume or more,
   further more preferably 50% by volume or more and 70% by volume or less, and
   further more preferably 50% by volume or more and less than 60% by volume.
[5] The method according to any one of [1] to [4], in which a concentration, in the aqueous solution, of the at least one selected from the group consisting of guanidine hydrochloride and guanidine thiocyanate is
   preferably 0.50 g/mL or more,
   and more preferably 0.55 g/mL or more.
[6] The method according to any one of [1] to [5], in which the treating the collection medium containing the skin surface lipids with the aqueous solution comprises
   preferably contacting the aqueous solution with the skin surface lipids contained in the collection medium,
   more preferably permeating the aqueous solution into the collection medium, and
   further more preferably permeating the aqueous solution into at least a portion containing the skin surface lipids in the collection medium.
[7] The method according to any one of [1] to [6], in which the collection medium is
   preferably a skin surface lipid-absorbent material, and
   more preferably a porous plastic film.
[8] The method according to any one of [1] to [7], in which the alcohol is preferably at least one selected from the group consisting of ethanol, isopropanol, and butanol.
[9] The method according to any one of [3] to [8], in which the drying the collection medium treated with the aqueous solution comprises
   preferably placing the collection medium under a dry environment in a closed space system, and
   more preferably causing the collection medium to coexist with a desiccant in a container.
[10] The method according to any one of [2] to [9], in which the storing the collection medium treated with the aqueous solution comprise storing the collection medium at a temperature of preferably 60°C or less, more preferably 40°C or less, further more preferably 37°C or less, further more preferably from 1 to 60°C, further more preferably from 1 to 40°C, further more preferably from 1 to 37°C, and further more preferably from 1 to 30°C.
[11] A kit for collecting skin surface lipid-derived RNA, comprising:
   a collection medium for collecting skin surface lipids; and
   an aqueous solution for treating the collection medium which comprises at least one selected from the group consisting of guanidine hydrochloride and guanidine thiocyanate and in which water or an alcohol aqueous solution is used as a solvent.
[12] The kit according to [11], preferably further comprising a container for storing the collection medium containing the skin surface lipids treated with the aqueous solution, and a desiccant.
[13] The kit according to [12], in which the container preferably holds the desiccant therein.
[14] The kit according to any one [11] to [13], in which the solvent is an alcohol aqueous solution, and an alcohol concentration in the alcohol aqueous solution is
   preferably 20% by volume or more,
   more preferably 20% by volume or more and 70% by volume or less,
   further more preferably 30% by volume or more,
   further more preferably 30% by volume or more and 70% by volume or less,
   further more preferably 50% by volume or more,
   further more preferably 50% by volume or more and 70% by volume or less,
   and further more preferably 50% by volume or more and less than 60% by volume.
[15] The kit according to any one of [11] to [14], in which a concentration, in the aqueous solution, of the at least one selected from the group consisting of guanidine hydrochloride and guanidine thiocyanate is
   preferably 0.50 g/mL or more, and
   more preferably 0.55 g/mL or more.
[16] The kit according to any one of [11] to [15], in which the collection medium is preferably a skin surface lipid-absorbent material, and
   more preferably a porous plastic film.
[17] The kit according to any one of [11] to [16], in which the alcohol is at least one selected from the group consisting of ethanol, isopropanol, and butanol.
[18] The kit according to any one of [12] to [17], in which the container is
   preferably a sealable container, and
   more preferably a container illustrated in Figure 1.
[19] A method for recovering skin surface lipid-derived RNA of a subject, comprising:
   providing, to a subject needing or desiring collection of skin surface lipid-derived RNA, the kit for collecting skin surface lipid-derived RNA, according to any one of [11] to [18], the kit comprising a collection medium for collecting skin surface lipids, and an aqueous solution for treating the collection medium,
   wherein the aqueous solution comprises at least one selected from the group consisting of guanidine hydrochloride and guanidine thiocyanate contained in which water or an alcohol aqueous solution is used as a solvent; and
   recovering, from the subject, the collection medium containing skin surface lipids collected from the subject,
   wherein the recovered collection medium containing the skin surface lipids has been treated with the aqueous solution in accordance with the method for preventing degradation of or for storing skin surface lipid-derived RNA according to any one of [1] to [10], and has been stored at ambient temperature after collecting the skin surface lipids of the subject.

### Examples

The present invention will now be described in more detail with reference to examples, and the present invention is not limited by these examples.

### Example 1 Prevention of RNA Degradation by Guanidine Salt

### (1) Preparation of Guanidine Hydrochloride Solution and Guanidine Thiocyanate Solution

Guanidine hydrochloride (FUJIFILM Wako Pure Chemical Corporation) was dissolved in a concentration of 6 M in a solvent of a 57% (v/v) ethanol aqueous solution. Guanidine thiocyanate (FUJIFILM Wako Pure Chemical Corporation) was dissolved in a concentration of 6 M in a solvent of a 57% (v/v) ethanol aqueous solution.

### (2) Preparation of Oil Absorbing Film

Four oil absorbing films (Oil Clear Film, Hakugen Earth Company Limited, using a porous polypropylene film of 3M, 25 x 40 mm) were used to collect SSL from the whole face of a subject. Separately, a RNeasy Mini Kit (Qiagen) was used to extract RNA from a human skin section, and then the resultant was dissolved in an RNA concentration of 1 µg/mL in a 57% (v/v) ethanol aqueous solution. The thus obtained human skin-derived RNA solution was evenly applied in an amount of 20 µL to each of the four oil absorbing films over the whole film to permeate. In the four oil absorbing films having the RNA applied thereto, the guanidine hydrochloride solution prepared in (1) was evenly applied to one film and the guanidine thiocyanate solution prepared in (1) was evenly applied to another film each in an amount of 20 µL over the whole film to permeate. No guanidine salt solution was applied to the other two oil absorbing films. Each of the four oil absorbing films was put in a screw tube (having a capacity of 20 mL), and then the resultant was stored at -80°C or room temperature (from 18 to 25°C) for 4 days (Table 1).

### (3) Evaluation of RNA Degradation Preventing Action

RNA was extracted from each of the oil absorbing films prepared and then stored in (2), and then the extracted RNA was converted to cDNA by reverse transcription, then the cDNA was subjected to PCR, and the resultant reaction product was purified. Specifically, RNeasy Mini Kit (Qiagen) was used to extract RNA from the oil absorbing film, the thus obtained RNA was converted to cDNA by reverse transcription using Super Script VILO cDNA Synthesis Kit (Thermo Scientific). The cDNA was subjected to PCR with Ion AmpliSeq Transcriptome Human Gene Expression Kit (Life Technologies Japan Ltd.), and the resultant reaction product was purified with magnetic beads (Ampure XP: Beckman Coulter, Inc.) to obtain 10 µL of a purified cDNA solution. A cDNA concentration in the purified solution was quantitatively determined with Agilent 4200 TapeStation (Agilent Technologies Japan, Ltd.) to obtain a relative cDNA concentration of each sample to a cDNA concentration in a sample stored at -80°C. As shown in Table 1, RNA degradation at room temperature was prevented by the guanidine salt treatment. Guanidine hydrochloride was superior to guanidine thiocyanate in the effect of preventing RNA degradation.

**[Table 1]**

| Sample No. | Guanidine Salt Treatment | Storage Condition | cDNA Concentration (pg/µL) | Relative cDNA Concentration (%) |
|---|---|---|---|---|
| 1 | not performed | -80°C | 63.8 | 100 |
| 2 | not performed | room temperature | 17.5 | 27.4 |
| 3 | Guanidine Hydrochloride | room temperature | 52.4 | 82.1 |
| 4 | Guanidine Thiocyanate | room temperature | 48.9 | 76.6 |

### Example 2 Prevention of RNA Degradation by Drying

### (1) Preparation of Desiccant

Three screw tubes (each having a capacity of 20 mL) were prepared, and then molecular sieves 13X 1/8 (FUJIFILM Wako Pure Chemical Corporation) were added to one of these to obtain a ratio between the screw tube capacity (mL) and the mass (g) of molecular sieves of 20:1. Specifically, 1.0 g of molecular sieves were added to the screw tube having a capacity of 20 mL.

### (2) Preparation of Oil Absorbing Film

Three oil absorbing films (Oil Clear Film, Hakugen Earth Company Limited, 25 x 40 mm) were used to collect SSL from the whole face of a subject. The human skin-derived RNA solution prepared in (2) of Example 1 was evenly applied in an amount of 20 µL over each of the whole films to permeate. The three oil absorbing films were respectively put in the screw tubes prepared in (1), and the resultant was stored at -80°C or room temperature (from 18 to 25°C) for 3 days (Table 2).

### (3) Evaluation of RNA Degradation Preventing Action

RNA was extracted through the same procedures as in (3) of Example 1 from each of the oil absorbing films prepared and then stored in (2), and then was converted to cDNA and subjected to PCR, and the resultant reaction product was purified, and then quantitatively determined to obtain a relative cDNA concentration of each sample to a cDNA concentration in a sample stored at -80°C. As shown in Table 2, RNA degradation at room temperature was prevented by drying.

**[Table 2]**

| Sample No. | Drying | Storage Condition | cDNA Concentratio n (pg/µL) | Relative cDNA Concentration (%) |
|---|---|---|---|---|
| 1 | not performed | -80°C | 57.6 | 100 |
| 2 | not performed | room temperature | 0 | 0 |
| 3 | Desiccant | room temperature | 17.5 | 30.4 |

### Example 3 Prevention of RNA Degradation by Guanidine Salt and Drying

### (1) Preparation of Guanidine Hydrochloride Solution

A guanidine hydrochloride solution was prepared through the same procedures as in (1) of Example 1.

### (2) Preparation of Desiccant

Three screw tubes were prepared through the same procedures as in (1) of Example 2, and then a desiccant was added to one of these.

### (3) Preparation of Oil Absorbing Film

Three oil absorbing films (Oil Clear Film, Hakugen Earth Company Limited, 25 x 40 mm) were used to collect SSL from the whole face of a subject. The human skin-derived RNA solution prepared in (2) of Example 1 was evenly applied in an amount of 20 µL over each of the whole films to permeate. In the three oil absorbing films having the RNA applied thereto, the guanidine hydrochloride solution prepared in (1) was evenly applied to one film in an amount of 20 µL over the whole film to permeate. The film having the guanidine hydrochloride solution applied thereto was put in the screw tube having the desiccant added thereto prepared in (2). No guanidine hydrochloride solution was applied to the other two oil absorbing films, and the oil absorbing films were respectively put in the screw tubes having no desiccant added thereto prepared in (2). Each of these samples was stored at -80°C or room temperature (from 18 to 25°C) for 3 days (Table 3).

### (4) Evaluation of RNA Degradation Preventing Action

RNA was extracted through the same procedures as in (3) of Example 1 from each of the oil absorbing films prepared and then stored in (3), and then was converted to cDNA and then subjected to PCR, and the resultant reaction product was purified, and then quantitatively determined to obtain a relative cDNA concentration of each sample to a cDNA concentration in a sample stored at -80°C. As shown in Table 3, RNA degradation at room temperature was remarkably prevented by employing the guanidine salt treatment and the drying together.

**[Table 3]**

| Sample No. | Treatment | Storage Condition | cDNA Concentration (pg/µL) | Relative cDNA Concentration (%) |
|---|---|---|---|---|
| 1 | not performed | -80°C | 545 | 100 |
| 2 | not performed | room temperature | 0 | 0 |
| 3 | Guanidine Hydrochloride + Desiccant | room temperature | 670 | 122.9 |

### Example 4 Prevention of RNA Degradation by Guanidine Salt Solution or Guanidine Salt-Alcohol Aqueous Solution and Drying

### (1) Preparation of Guanidine Hydrochloride Solution

Guanidine hydrochloride (FUJIFILM Wako Pure Chemical Corporation) was dissolved in a concentration of 6 M in a solvent of a 30% (v/v) ethanol aqueous solution or water (UltraPure DNase/RNase-Free Distilled Water: Thermo Fisher Scientific K.K.).

### (2) Preparation of Desiccant

Four screw tubes (each having a capacity of 20 mL) were prepared, and then molecular sieves 13X 1/8 (FUJIFILM Wako Pure Chemical Corporation) were added to two of these to obtain a ratio between the screw tube capacity (mL) and the mass (g) of molecular sieves of 20:1. Specifically, 1.0 g of molecular sieves were added to the screw tube having a capacity of 20 mL.

### (3) Preparation of Oil Absorbing Film

Four oil absorbing films (Asemo Toreru Oil Clear Film (Oil Clear Film capable of removing sweat), Hakugen Earth Company Limited, using a porous polypropylene film of 3M, 25 x 40 mm) were used to collect SSL from the whole face of a subject. Separately, a RNeasy Mini Kit (Qiagen) was used to prepare Human HeLa Cell Total RNA (Takara Bio Inc.) in an RNA concentration of 1 µg/mL with UltraPure DNase/RNase-Free Distilled Water (Thermo Fisher Scientific K.K.). The thus obtained HeLa Cell-derived RNA solution was evenly applied in an amount of 20 µL over each of the whole films to permeate. In the four oil absorbing films having the RNA applied thereto, the guanidine hydrochloride solution prepared in (1) with a 30% (v/v) ethanol aqueous solution used as a solvent was evenly applied to one film, and the guanidine hydrochloride solution prepared with water used as a solvent was evenly applied to another film each in an amount of 20 µL over the whole film to permeate. These two films having the guanidine hydrochloride solution applied thereto were respectively put in the screw tubes having the desiccant added thereto prepared in (2). No guanidine hydrochloride solution was applied to the other two oil absorbing films, and these oil absorbing films were respectively put in the screw tubes having no desiccant added thereto prepared in (2). Each of these samples was stored at -80°C or 37°C for 3 days (Table 4).

### (4) Evaluation of RNA Degradation Preventing Action

RNA was extracted through the same procedures as in (3) of Example 1 from each of the oil absorbing films prepared and then stored in (3), and then was converted to cDNA and then subjected to PCR, and the resultant reaction product was purified, and then quantitatively determined to obtain a relative cDNA concentration of each sample to a cDNA concentration in a sample stored at -80°C. As shown in Table 4, RNA degradation at 37°C was remarkably prevented by the guanidine salt treatment and the drying.

**[Table 4]**

| Sample No. | Drying | Storage Condition | cDNA Concentration (pg/µL) | Relative cDNA Concentration (%) |
|---|---|---|---|---|
| 1 | not performed | -80°C | 3420 | 100 |
| 2 | not performed | 37°C | 229 | 6.7 |
| 3 | Guanidine Hydrochloride (30%EtOH) + Desiccant | 37°C | 3210 | 93.9 |
| 4 | Guanidine Hydrochloride (H₂O) + Desiccant | 37°C | 2760 | 80.7 |

### Example 5 Prevention of RNA Degradation by Guanidine Salt and Drying in Storage at 60°C

### (1) Preparation of Guanidine Hydrochloride Solution

A guanidine hydrochloride solution was prepared through the same procedures as in (1) of Example 1.

### (2) Preparation of Desiccant

Three screw tubes were prepared through the same procedures as in (1) of Example 2, and then a desiccant was added to one of these.

### (3) Preparation of Oil Absorbing Film

Three oil absorbing films (Oil Clear Film, Hakugen Earth Company Limited, 25 x 40 mm) were used to collect SSL from the whole face of a subject. The HeLa Cell-derived RNA solution prepared in (3) of Example 4 was evenly applied in an amount of 20 µL over each of the whole films to permeate. In the three oil absorbing films having the RNA applied thereto, the guanidine hydrochloride solution prepared in (1) was evenly applied to one film in an amount of 20 µL over the whole film to permeate. The film having the guanidine hydrochloride solution applied thereto was put in the screw tube having the desiccant added thereto prepared in (2). No guanidine hydrochloride solution was applied to the other two oil absorbing films, and the oil absorbing films were respectively put in the screw tubes having no desiccant added thereto prepared in (2). Each of these samples was stored at -80°C or 60°C for 3 days (Table 5).

### (4) Evaluation of RNA Degradation Preventing Action

RNA was extracted through the same procedures as in (3) of Example 1 from each of the oil absorbing films prepared and then stored in (3), and then was converted to cDNA and then subjected to PCR, and the resultant reaction product was purified, and then quantitatively determined to obtain a relative cDNA concentration of each sample to a cDNA concentration in a sample stored at -80°C. As shown in Table 5, RNA degradation in storage at 60°C was remarkably prevented by the guanidine salt treatment and the drying.

**[Table 5]**

| Sample No. | Drying | Storage Condition | cDNA Concentration (pg/µL) | Relative cDNA Concentration (%) |
|---|---|---|---|---|
| 1 | not performed | -80°C | 2470 | 100 |
| 2 | not performed | 60°C | 161 | 6.5 |
| 3 | Guanidine Hydrochloride + Desiccant | 60°C | 2580 | 104.5 |

## Claims

1. A method for preventing degradation of skin surface lipid-derived RNA, comprising:
treating a collection medium containing skin surface lipids with an aqueous solution which comprises at least one selected from the group consisting of guanidine hydrochloride and guanidine thiocyanate and in which water or an alcohol aqueous solution is used as a solvent.

2. A method for storing skin surface lipid-derived RNA, comprising:
treating a collection medium containing skin surface lipids with an aqueous solution which comprises at least one selected from the group consisting of guanidine hydrochloride and guanidine thiocyanate and in which water or an alcohol aqueous solution is used as a solvent; and
storing the treated collection medium.

3. The method according to claim 1 or 2, further comprising drying the collection medium treated with the aqueous solution.

4. The method according to any one of claims 1 to 3, wherein the solvent is an alcohol aqueous solution having an alcohol concentration of 20% by volume or more and 70% by volume or less.

5. The method according to any one of claims 1 to 4, wherein a concentration, in the aqueous solution, of the at least one selected from the group consisting of guanidine hydrochloride and guanidine thiocyanate is 0.50 g/mL or more.

6. The method according to any one of claims 1 to 5, wherein the treating comprises permeating the aqueous solution in the collection medium containing skin surface lipids.

7. A kit for collecting skin surface lipid-derived RNA, comprising:
a collection medium for collecting skin surface lipids; and
an aqueous solution for treating the collection medium which comprises at least one selected from the group consisting of guanidine hydrochloride and guanidine thiocyanate and in which water or an alcohol aqueous solution is used as a solvent.

8. The kit according to claim 7, further comprising a container for storing the collection medium containing skin surface lipids treated with the aqueous solution, and a desiccant.

9. The kit according to claim 8, wherein the container holds the desiccant therein.

10. The kit according to any one of claims 7 to 9, wherein the solvent is an alcohol aqueous solution having an alcohol concentration of 20% by volume or more and 70% by volume or less.

11. The kit according to any one of claims 7 to 10, wherein a concentration, in the aqueous solution, of the at least one selected from the group consisting of guanidine hydrochloride and guanidine thiocyanate is 0.50 g/mL or more.

12. A method for recovering skin surface lipid-derived RNA of a subject, comprising:
providing, to a subject needing or desiring collection of skin surface lipid-derived RNA, the kit for collecting skin surface lipid-derived RNA according to any one of claims 7 to 11, the kit comprising a collection medium for collecting skin surface lipids, and an aqueous solution for treating the collection medium,
wherein the aqueous solution comprises at least one selected from the group consisting of guanidine hydrochloride and guanidine thiocyanate in which water or an alcohol aqueous solution is used as a solvent;
recovering, from the subject, the collection medium containing skin surface lipids collected from the subject,
wherein the recovered collection medium containing the skin surface lipids has been treated with the aqueous solution in accordance with the method for preventing degradation of or for storing skin surface lipid-derived RNA according to any one of claims 1 to 6, and has been stored at ambient temperature after collecting the skin surface lipids of the subject.
